# EUROPEAN PATENT APPLICATION

(11) **EP 1 033 402 A1**
(43) Date of publication of application: **06.09.2000**
(21) Application number: 98951686.9
(22) Date of filing: 04.11.1998
(51) Int. Cl.: C12N 15/12, C07K 14/47, C07K 16/18

(54) **p16-BINDING PROTEINS, GENE THEREOF, AND ANTIBODY THEREAGAINST**

(30) Priority: 05.11.1997 JP 30256497
(71) Applicant: Sumitomo Electric Industries, Ltd., Osaka-shi, Osaka 541-0041 (JP)
(72) Inventor: NAKAMURA, Takeshi, Sumitomo Electr.Industries,Ltd., Yokohama-shi Kanagawa 244-8588 (JP); HARA, Eiji, Paterson Inst. for Cancer Research, Manchester (GB)
(74) Representative: Baldock, Sharon Claire
(86) International application number: JP9804972
(87) International publication number: WO9923218

(57) **Abstract**

A novel protein p16BP1 derived from human which binds to a tumor suppressor gene product p16, its variant protein, a polynucleotide encoding the same, and an antibody which recognizes said proteins, are provided.

## Description

### Technical Field

The present invention relates to a novel protein p16BP1 which binds to a tumor suppressor gene product p16, and relates to a gene encoding the sama.

### Background Art

A tumor suppressor gene product p16 is a cycline-dependant kinase inhibitor (CDKI) which specifically binds to cycline-dependant kinases CDK4 and CDK6 and inhibits their activities. 7 types of CDKI in mammal cells including p16 have been cloned so far, and only p16 is found to have abnormality with high frequency in cancer cells.

The knockout mice with respect to p16 have much cancers in various tissues, and the primary culture fibroblast derived from their embryos becomes immortal (i.e., it can permanently proliferate) without reaching senescence, suggesting that p16 is a tumor suppressor gene product which inhibits malignant alteration.

On the other hand, the expression level of p16 gene in normal cells is extremely low but increases as the number of cell division increases, suggesting that p16 can be a gene product which induces cell aging.

As described above, p16 is considered to be a gene product having a function of preventing malignant alteration by inducing cell aging.

Structurally, p16 has 4 ankyrin repeat domains which are important for protein-protein interaction. Accordingly, p16 is supposed to bind to proteins other than CDK4 and CDK6 mentioned above. However, such proteins have not yet been isolated.

### Disclosure of the Invention

Proteins binding to p16 are considered to directly concern the control of extremely important phenomena including malignant alteration or cell aging by controlling a function of p16.

An object of the present invention is to isolate a novel p16-binding protein and determine its amino acid sequence. Another object of the present invention is to provide a polynucleotide which encodes the aforementioned amino acid sequence. Further another object of the present invention is to provide an antibody which recognizes the aforementioned novel p16-binding protein.

These objects have been accomplished by the present invention mentioned below provided by the present inventors.

Thus, the present invention provides human p16BP1 (the amino acid sequence is represented by SEQ ID No. 1 in the sequence listing) which is a novel protein binding to human p16.

The present invention also provides a protein of human p16BP1 variant which has an amino acid sequence in which one or more amino acids are deleted, one or more amino acids are substituted with another amino acid, and/or one or more amino acids are added in the human p16BP1, that is the amino acid sequence of SEQ ID No. 1 in the sequence listing, and is capable of binding to human p16.

Another aspect of the present invention provides a polynucleotide which encodes the aforementioned human p16BP1 or human p16BP1 variants. Typical polynucleotides are DNA or RNA. In the present specification, a term "polynucleotide" is used as that having a widest sense involving those chemically modified which do not exist in nature.

A cDNA which encodes naturally occurring human p16BP1 (hereinafter referred to as "human p16BP1 cDNA") has a base sequence of SEQ ID No. 2 in the sequence listing.

Also, the present invention provides an antisense polynucleotide comprising the base sequence of the antisense strand of the aforementioned polynucleotide, and an derivative of the antisense polynucleotide. The antisense polynucleotide is involved in polynucleotide. In the present specification, the term antisence polynucleotide is used as a lower conception of polynucleotide especially in the case where it is clearly expressed that a polynucleotide has an antisense strand.

Also, the present invention provides a part of the aforementioned polynucleotide or the aforementioned antisense polynucleotide, which consists of consecutive 12 or more bases.

The present invention also provides the aforementioned polynucleotide and the aforementioned antisense polynucleotide, which are chemically modified. As described above, the term "polynucleotide" used in the present specification involves chemically modified polynucleotides, and the term chemically modified polynucleotide is used as a lower conception of polynucleotide especially in the case where it is clearly expressed that a polynucleotide is chemically modified.

A further another aspect of the present invention provides a method for obtaining cDNA which is a homologue of human p16BP1 cDNA having a base sequence of SEQ ID No. 2 in the sequence listing, wherein the aforementioned polynucleotide or antisense polynucleotide is used as a probe and an cDNA capable of hybridizing with the probe is obtained from cDNA library of animals other than human. The length of the cDNA is almost the same as that of human p16BP1 cDNA, that is 650-780 bp.

Also, the present invention provides cDNA obtained by the aforementioned method.

Also, the present invention provides a protein which is a homologue of human p16BP1, that is a protein of p16BP1 of the animals other than human and has an amino acid sequence encoded by the cDNA obtained by the aforementioned method. The protein has a molecular weight of approximately 34 kD and a biological function of binding to p16.

In the present specification, an assembly of protein comprising human p16BP1 and its homologues in the other animals is referred to as "p16BP1". Also, a protein which is an element of the assembly is also referred to as p16BP1 when the animal from which the protein is derived is not specified.

Also, the present invention provides an antibody which recognizes human p16BP1, human p16BP1 variants, or p16BP1 of animal other than human.

### Brief Description of Drawings

Fig. 1 is a photograph of result of electrophoresis wherein it is confirmed by an in vitro binding assay that human p16BP1 binds to human p16.
Fig. 2 is a diagram representing the expression of human p16BP1 mRNA in various normal tissues of the human by comparison.
Fig. 3 is a diagram representing the change of the expression level of human p16BP1 mRNA with the progression of the cell cycle.
Fig. 4 is a photograph of result of electrophoresis which represents the expression of p16BP1 with time determined by the Western blotting using anti-p16BP1 antibody and anti-cyclin A antibody.
Fig. 5 is a photograph of result of electrophoresis where the interactions between p16BP1 and p16, p18, p19 or p27 are examined. In the figure, the result in the upper column represents the case where the proteins are mixed and the resultant sample is directly analyzed, and the results in the lower column represents the case where the sample is analyzed after immunoprecipitation with an antibody against p16BP1.
Fig. 6 is a photograph of result of electrophoresis where the interactions of p16BP1, p16, and CDK4 are examined.
Fig. 7 is a photograph of result of electrophoresis where, as to the mixed samples of Cyclin D1-CDK4 complex with p16, p16BP1, Id1, a kinase activity is examined by using GST-Rb protein as a substrate.
Fig. 8 is a photograph of result of electrophoresis where the effect of p16BP1 on the inhibitory action of p16 to the binding of Cyclin D1 and CDK4 is examined.
Fig. 9 is a photograph of result of electrophoresis where the effect of p16BP1 on the binding of Cyclin D1 and CDK4 is examined. In the figure, the upper 3 columns represent the result wherein the amounts of p16BP1, Cyclin D1 and CDK4 in the cell extract solution are respectively confirmed by Western blotting. The lowest column represents the result where the cell extract solution was subjected to immunoprecipitation with Cyclin D1 antibody and then to Western blotting with CDK4 antibody.
Fig. 10 is a photograph of result of electrophoresis where the expression levels of p16BP1 in the stable transformed cells having different expression levels of p16BP1 which were established in order to examine the action of p16BP1 on cell growth of rat fibroblast, are examined. In the figure, F-2 represents the result of cells expressing p16BP1 in low level, F-11 represents that of cells expressing p16BP1 in high level, and N-2 represents that of cells transformed with a vector only.
Fig. 11 is a diagram which represents the result where the extracts prepared from the stable transformed cell shown in Fig. 10 are analyzed by immunoblotting using an antibody against p16BP1. In the figure, □, ●, and ○ represent the result of N-2, F-2, and F-11, respectively.

### Best Mode for Carrying Out the Invention

As a probe used for obtaining p16BP1 cDNA in each animal from the animal cDNA library, human p16BP1 cDNA can be used. Most preferably, p16BP1 cDNA of mammals can be obtained from the mammal cDNA library. Alternatively, as the probe, RNA having a base sequence corresponding to the aforementioned human p16BP1 cDNA can be used.

A method for obtaining p16BP1 cDNA from cDNA library of each animal is exemplified below.

### 1. Preparation of cDNA library

cDNA library of a desired vertebrate animal is prepared from mRNA of the animal. Alternatively, commercial cDNA library may be used. Approximately 10⁶ plaques are prepared on a NZY agar medium, and the cDNA library is fixed on a nitrocellulose membrane according to the method described on pages 9.38-9.41 of "Molecular Cloning Second Edition" (1989, Cold Spring Harbor Laboratory Press). Then, the membrane is washed with 2 × SSC at 60°C.

### 2. Isolation of p16BP1 cDNA

By using, for example, a cDNA having the base sequence of SEQ ID No. 2 in the sequence listing as a probe, a full length p16BP1 cDNA can be obtained from the abovementioned cDNA library by the following procedures.
1) A probe DNA is labeled. For example, Megaprime Labeling Kit (RTM, Amersham) can be used.
2) 150 µl of the labeled DNA probe fraction obtained above is hybridized with cDNA probe of the plaque fixed on the membrane under the following conditions.
   ① Prehybridization
      6 × SSC
      5 × Denhaldt's
      0.05% sodium pyrophosphate
      100 µg/ml denatured herring sperm DNA
      0.5% SDS
      Total amount of solution 50 ml
      Reaction temperature 37°C
      Reaction time 1 hour
   ② Hybridization
      6 × SSC
      1 × Denhaldt's
      0.05% sodium pyrophosphate
      100 µg/ml denatured herring sperm DNA
      1 × 10⁶ cpm/ml cDNA probe
      Total amount of solution 50 ml
      Reaction temperature 42°C
      Reaction time 18 hours
6) The nitrocellulose membrane after hybridization is washed under the following conditions once each.
   ① 6 × SSC, 0.1% SDS 500 ml.
      Temperature 40°C
      Time 20 minutes
   ② 3 × SSC, 0.1% SDS 500 ml
      Temperature 42°C
      Time 20 minutes
7) The washed nitrocellulose membrane is exposed to light on a KODAK XAR5 (RTM) film (Kodak) at -80°C overnight to give an autoradiograph.
8) From the resultant autoradiograph, the positions of the positive plaques are determined, and the corresponding plaques on the agar are collected into SM solution.
9) The collected plaques are subjected to plaque formation again on the NZY agar medium according to the conventional method, and fixed on a nitrocellulose membrane.
10) The processes of 5) to 9) are repeated 3 times. The resultant single positive plaque is collected and suspended in 100 µl of SM solution, and the phage is stabilized. From the plaque, p16BP1 cDNA can be isolated.

### 3. Large scale preparation of p16BP1 cDNA

1) 50 µl of the phage solution of the plaque suspended in SM solution is mixed with 20 µl of Y1090r-E. coli, and allowed to stand at 37°C for 15 minutes.
2) After that, the solution mixed in step 1) is transferred to 10 ml of the NZY medium containing 100 µg/ml of ampicillin, and cultured at 37°C for 6 hours.
3) Centrifugation is carried out at 8,000 rpm for 5 minutes, and the supernatant is collected.
4) To the supernatant, 1 ml of 5M NaCl and 1.1 g of polyethyleneglycol 6000 are added and dissolved.
5) The solution is placed on ice for 1 hour and centrifuged at 10,000 rpm at 4°C for 20 minutes.
6) The precipitate is collected and suspended in 700 µl of SM solution.
7) 500 µl of chloroform is added and the mixture is stirred to dissolve the residual E. coli.
8) Centrifugation is carried out at 5,000 rpm for 10 minutes, and the aqueous layer is collected.
9) To the aqueous layer, 1 µl of 1 mg/ml RNaseA (Sigma) and 1 µl of 5 mg/ml DNaseI (Sigma) are added, and the mixture was allowed to stand at 37°C for 1 hour. Then, 600 µl of 20% polyethyleneglycol 6000 (0.8M NaCl) is added, and the resultant mixture is allowed to stand on ice for 30 minutes.
10) Centrifugation is carried out at 15,000 rpm at 4°C for 20 minutes, and the precipitate is collected.
11) To the precipitate, 500 µl of SM solution, 50 µl of 5M NaCl and 50 µl of 0.5M EDTA are added, and 400 µl of phenol is further added, and the mixture is stirred to dissolve the phage and release cDNA.
12) The resultant solution is centrifuged at 15,000 rpm at room temperature for 5 minutes, and the aqueous layer is collected. 1 ml of ethanol is added thereto, and centrifugation is carried out at 15,000 rpm for 20 minutes, and the liquid layer is discarded.
13) The precipitate is washed with 1 ml of 70% ethanol and dissolved in 100 µl of TE solution (10 mM Tris-Cl (pH 8.0), 1 mM EDTA). The DNA solution can be obtained thereby.

### 4. Determination of base sequence of p16BP1 cDNA

All the base sequence of p16BP1 cDNA is determined by the die-terminator method. An auto-sequencer can be used.

### 5. Determination of amino acid sequence

From the base sequence determined in step 4. above, the amino acid sequence of p16BP1 can be determined. For example, a commercial program (e.g., GENETYX (RTM) -CD program Ver. 34 (Software Development inc.)) can be used.

### 6. Preparation of transformant

p168P1 cDNA is obtained according to the above mentioned method. This p16BP1 cDNA can be inserted in a vector and introduced to a proper host to prepare a transformant. As a vector, for example, pGEX-5T is mentioned. As an example of the host, E. coli JM109 is mentioned. By culturing this transformant in a proper medium, a protein p16BP1 of the present invention can be expressed, and by using a means of separation and purification well-known by a person with ordinary skill in the art, the purified protein can be obtained. The protein p16BP1 of the present invention prepared thereby has a molecular weight of 34 kD and a biological action of binding to p16.

By natural mutation or artificial mutation (e.g., a method described in Molecular Cloning 2nd Edition (Cold Spring Harbor Laboratory Press, 1989) on pages 15.1-15.113), a polynucleotide can be varied without altering main functions of the polypepetide (including protein) encoded by the polynucleotide. For example, by using this method, as to human p16BP1 of the present invention, a protein having an amino acid sequence in which one or more, preferably one or several amino acids are deleted, one or more, preferably one or several amino acids are substituted, and/or one or more, preferably one or several amino acids are added in the amino acid sequence of SEQ ID No. 1 in the sequence listing, and having the substantially same biological function as human p16BP1 (e.g., action of binding to human p16),that is a human p16BP1 variant capable of binding to human p16, can be prepared. It should be understood that this human p16BP1 variant is involved in the scope of the present invention.

The amino acid sequence of the human p16BP1 variant of the present invention can be determined from the base sequence of cDNA which encodes the variant. For example, a commercial program (e.g., GENETYX (RTM) -CD program Ver. 34 (Software Development Inc.)) can be used.

Due to degeneracy of genetic code, at least a part of the bases of the base sequence of the polynucleotide can be substituted with another kind of base without altering the amino acid sequence of polypeptide (including protein) generated from polynucleotide. Accordingly, the polynucleotide which encodes p16BP1 of the present invention includes all the pattern of degeneracy.

The present invention relates to an antisense polynucleotide having the base sequence of the antisense strand of the aforementioned polynucleotide which encodes human p16BP1, and to its derivative. The antisense polynucleotide means that capable of hybridizing to the polynucleotide which encodes human p16BP1, and if the polynucleotide to which the antisense polynucleotide hybridizes is a code region, the antisense polynucleotide can inhibit biosynthesis of the polypeptide encoded by the polynucleotide.

The anitisense polynucleotide for inhibiting biosynthesis of polypeptide preferably has 12 or more bases. On the other hand, in order to incorporate full length antisense polynucleotide into cells, too long one is not proper. In case of incorporating antisense polynucleotide into cells to inhibit biosynthesis of p16BP1, an antisense polynucleotide having 12 to 30 bases, preferably 15 to 25 bases, more preferably 18 to 22 bases is preferably used. The antisense polynucleotide can also be used as a probe.

The antisense polynucleotide of the present invention or a part of it involves those where plural nucleotides consisting of base, phosphate and sugar are bound , which may not exist in nature. Typical antisense polynucleotides are antisense DNA and antisense RNA.

By using well-known antisense techniques, various antisense polynucleotide derivatives having high binding ability with desired DNA or mRNA, high tissue selectivity, high cell permeability, high nuclease resistance, and high intracellular stability can be prepared, and all of these derivatives are involved in the antisense polynucleotide of the present invention.

In view of easiness of hybridization, in general, it is desired to design an antisense polynucleotide having a complementary base sequence to the base sequence of the region forming a stem loop, or its derivatives. The antisense polynucleotide of the present invention and its derivatives are capable of forming a stem loop, if necessary.

Antisense polynucleotides having complementary sequence to the sequence in the vicinity of the translation initiation codon, at a ribosome binding site, the capping site, or a splice site, can be generally expected to have a high expression inhibitory effect. Accordingly, the antisense polynucleotides of the present invention or its derivatives which contain a gene encoding p16BP1 or a complementary sequence to the sequence in the vicinity of the translation initiation codon, at a ribosome binding site, the capping site, a splice site of mRNA, are expected to have a high expression inhibitory effect.

The currently generally known derivatives are preferably those where at least one of nuclease resistance, tissue selectivity, cell permeability and binding ability is enhanced. Particularly preferred derivatives are those having a phosphorothioate bond as a skeletal structure. The polynucleotide of the present invention and its derivatives involve derivatives having these functions or structures.

If the antisense polynucleotide is a natural type, the antisense polynucleotide of the present invention can be prepared by synthesis using a chemical synthesizer, or PCR method using a gene which encodes human p16BP1 as a template. Also, some of the derivatives such as methyl phosphonate type and phosphorothioate type can be chemically synthesized. In this case, the desired antisense polynucleotide or its derivatives can be obtained by carrying out the operations according to the manual attached to the chemical synthesizer, and purifying the resultant product by HPLC using reversed phase chromatography etc.

The polynucleotide which encodes human p16BP1 of the present invention, its antisense polynucleotide, or a polynucleotide consisting of a part thereof (polynucleotide having a base sequence of consecutive 12 or more bases), can be used as a probe for the screening of p16BP1 cDNA from cDNA library. In this case, those having GC content of 30-70% can preferably be used. Moreover, polynucleotides having a base sequence of consecutive 15 or more bases are particularly preferred. The polynucleotide used as a probe may be a derivative. Usually, sequences having not less than the above-mentioned number of base are recognized as those having specificity. As a cDNA library used in screening using such a probe, those prepared from mRNA can preferably be used. Also, a group of cDNAs selected from these cDNA libraries by random sampling can be used as a sample of screening. Alternatively, commercially available library may be used.

The polynucleotide which encodes human p16BP1 of the present invention, its antisense polynucleotide, or a polynucleotide consisting of a part thereof (polynucleotide having a base sequence of consecutive 12 or more bases) can be used as a probe. By performing northern blot hybridization for mRNA derived from each tissue using the polynucleotide as a probe, the tissue which expresses mRNA derived from human p16BP1 gene can be identified.

In chemical synthesis of DNA or RNA, a means of chemical modification such as that of methylation or biotinylation of side chains, or that of substituting oxygen of the phosphate group with sulfur, has been well-known. For example, if chemical modification is performed in chemical synthesis of DNA of SEQ ID No. 2 in the sequence listing, polynucleotide different from this DNA itself can be synthesized.

Also, cDNA obtained from a cDNA library may be labeled with a radioisotope.

Accordingly, DNA and RNA of the present invention includes the abovementioned DNA, RNA or antisense polynucleotide which are chemically modified. The chemically modified DNA or RNA is capable of exhibiting a function of encoding protein and a function of a probe, and the chemically modified antisense polynucleotide is capable of exhibiting a function of probe and a function of inhibiting biosynthesis of protein.

By using a well-known method, a transformant can be obtained by introducing a plasmid to a suitable host such as E. coli. It is possible to culture the resultant transformant to amplify the gene or express the protein, and prepare p16BP1. Similarly, it is possible to culture a transformant introduced with DNA which encodes a p16BP1 variant, and prepare a p16BP1 variant.

p16BP1 or p16BP1 variants of the invention can be purified by collecting a culture containing p16BP1 or p16BP1 variants produced by the transformant, and performing procedures such as condensation, solubilization, dialysis, various chromatography, etc., if necessary.

There are many textbooks concerning culture of tranformants, and according to well-known methods, p16BP1 or p16BP1 variants can be expressed on the basis of the base sequence described in the present invention. In this case, as a host, any of bacteria such as E. coli, yeast and animal cells can be used, and animal cells are particularly preferred. In order to introduce the gene into cells, a ribosome method, an electroporation method and the like can be used.

Purification method for purifying p16BP1 or p16BP1 variants from the resultant culture includes immunoprecipitation, salting out, ultrafiltration, isoelectric precipitation, gel filtration, electrophoresis, various affinity chromatography such as ion-exchange chromatography, hydrophobic chromatography and antibody chromatography, chromatofocusing, adsorption chromatography, reversed phase chromatography and the like, and any method can be suitably selected.

In the preparation step, p16BP1 or p16BP1 variants to be prepared may be generated by a transformant as a fusion peptide with another polypeptide. In this case, it is necessary to cut out p16BP1 or p16BP1 variants in the purification step by treating the fusion peptide with a chemical substance such as cyanogen bromide or an enzyme such as protease.

By immunizing an animal other than human and other than animals from which p16BP1 derives with p16BP1, p16BP1 variants or polypeptide having an amino acid sequence specific to then, an antibody recognizing p16BP1 (p16BP1 antibody) or an antibody recognizing p16BP1 variants (p16BP1 variant antibody) can be obtained, respectively. Recognition of p16BP1 or p16BP1 variants by the p16BP1 antibody or p16BP1 variant antibody can be confined by western blotting, ELISA, immunostaining (e.g., determination with FACS), and the like.

Use of a part of protein as an immunogen, especially those comprising a part of the protein which are bound to another carrier protein such as bovine serum albumin, is a conventional method. A part of the protein may be synthesized by, for example, using a peptide synthesizer. As a part of protein, those having 8 or more amino acid residues are preferred.

With respect to the substances which have antigenecity, if polyclonal antibody is obtained by immunosensitization, it is well-known to generate monoclonal antibody by hybridoma by using lymphocytes of the immunized animal. Accordingly, the antibody of the present invention involves monoclonal antibody in its scope.

The antibody of the present invention also involves active fragments of the antibody. The active fragment means a fragment of antibody having antigen-antibody reaction activity, and includes concretely F(ab')₂, Fab', Fab, Fv, and the like. For example, when the antibody of the present invention is digested with pepsin, F(ab')₂ is obtained, and when digested with papain, Fab is obtained. When F(ab')₂ is reduced with a reagent such as 2-mercaptoethanol and alkylated with acetic monoiodide, Fab' is obtained. Fv is an univalent antibody-active fragment obtained by binding a heavy chain variable region and a light chain variable region by using a linker. Moreover, chimera antibody can be obtained by maintaining these active fragments and substituting the other region with a fragment of the other animals. Any of these active fragments and chimera antibody is involved in the scope of the present invention.

The antibody of the present invention can be used for purification and detection of p16BP1, and utilized for elucidation of functions such as expression of p16BP1, control of the cell cycle, or study for suppression of cancer, as well as, treatment or diagnosis of diseases on the basis of these findings.

For detection of p16BP1, a method using antibody and a method utilizing enzyme reaction may be used.

A method using antibody includes, concretely, ① a method for detecting p16BP1 by using labeled p16BP1 antibody, and ② a method for detecting p16BP1 by using p16BP1 antibody and labeled secondary antibody of the antibody. As a label, for example, radioisotope (RI), enzyme, avidin or biotin, or fluorescent substances (FITC, rhodamine, etc.) may be utilized.

A method utilizing enzyme reaction includes, for example, identification of immunoreactive molecules by ELISA, immune agglutination, western blotting, or flow cytometry, and a similar method to them.

### Examples

The present invention will be further explained with reference to the following examples. However, the present invention is not limited to these examples.

### 〈Example 1〉 Cloning of human p16BP1 cDNA

The inventors of the present invention used yeast two hybrid screening system to obtain a novel protein which binds to human p16. This method comprises fusing two gene products to the DNA binding site and the transcription activating site of a transcription factor respectively, and detecting the interaction of them using transcription activity as an indicator.

The yeast two hybrid screening was performed by using MATCHMAKER (RTM) Two-Hybrid System 2 (a kit of Clontech, catalogue No. K1604-1) according to the attached manual. As a bait (a bait for catching binding protein), DNA obtained by inserting human p16 cDNA into the cloning site of plasmid pAS1 was used. The library used for the screening was a cDNA library derived from HeLa cells (Clontech).
(1) 5×10⁵ clones from cDNA library derived from HeLa cells were screened using as an indicator the fact that it can be cultured in a His-free medium by MATCHMAKER Two-Hybrid System 2 and that β-galactosidase is generated, and three positive clones (those recognized to bind to the bait of p16) were obtained.
(2) The plasmids containing these clones were transferred from yeast to E. coli.
   ① The positive clones were inoculated in 5 ml of the SD-LEU medium and cultured for 24 hours.
   ② The resultant culture (1.5ml) was placed in a micro-centrifugation tube, and centrifugation was performed at 10,000 rpm for 10 seconds to collect the bacterial body.
   ③ The supernatant was discarded, and the bacterial body was suspended in 0.1 ml of TELT solution.
   ④ A mixture of phenol/chloroform (1/1) (0.1 ml) and about 0.2 g of acid-washed glass beads were added, and the resultant mixture was subjected to vigorous voltex for 2 minutes.
   ⑤ Centrifugation was performed at 12,000 rpm for 1 minute, the upper layer was separated to place in another tube while not taking the middle layer, and 0.2 ml of ethanol was added for ethanol precipitation to collect DNA. The resultant DNA was washed with 70% ethanol, fully dried, and dissolved in 30 µl of TE buffer.
   ⑥ The DNA solution prepared in step ⑤ was introduced into E. coli HB101 strain by electroporation. E. coli was cultured in M9 medium, and plasmid was collected from the resultant transformant, digested with a restriction enzyme to confirm that the DNA is derived from pGAD-GH.
(3) The plasmid was prepared by using a kit of QIAwell 8 plus (RTM, Qiagen) according to the instruction.
(4) The base sequences of the insert in this plasmid were determined by using DNA sequencer 373A (Parkinelmer) and Taq cycle sequencing method (Biotechniques, 7, 494-499). One of them was CDK4. The other two were identical unknown gene, and a protein having the amino acid sequence encoded by this cDNA was termed human p16 binding protein 1, that is, human p16BP1. The aforementioned cDNA was termed human p16BP1 cDNA.

From the analysis result of base sequences of cDNA, of the resultant human p16BP1, 711 bases corresponding to the open reading frame are shown as SEQ ID No. 2 in the sequence listing.

The amino acid sequence of human p16BP1 encoded by this base sequence is shown as SEQ ID No. 1 in the sequence listing. Human p16BP1 has not been recognized to have significant homology with any known protein at present. The molecular weight of human p16BP1 is 34 kD.

The aforementioned E. coli which incorporated cDNA of human p16BP1 was termed "pBS-SEI1" and deposited in the National Institute of Bioscience and Human-Technology Agency of Industrial Science and Technology (1-1-3, Higashi, Tsukuba-shi, Ibaraki-ken, Japan) on October 28, 1997 (the accession number: FERM P-16492). After that, this deposit was transferred to the international deposit on the basis of the Budapest treaty dated October 22, 1998 (the accession number: FERM BP-6554).

### 〈Example 2〉 Detection of human p16 and human p16BP1 by in vitro binding assay

According to the method described on page 1587 of Oncogene, Vol. 11, in vitro binding assay was performed by using human p16 and human p16BP1 obtained by in vitro transcription/translation from p16 cDNA and p16BP1 cDNA, respectively. The outline of this method is shown below.
1) By using TNT expression system (Promega) and T7 polymerase, ³⁵S-methionine labeled proteins (p16BP1, cdk4, ΔE12 and p16) were synthesized by transcription and translation of the plasmid DNA.
2) 5 µl of p16 was mixed with 5 µl of one of the other three proteins, and the mixture was incubated at 30°C for 30 minutes.
3) Then, the mixture was diluted with 1 ml of ice-cold NP40 buffer (50 mM Tris-HCl (pH 7.5), 0.5 mM NaCl, 1% NP40, 3% BSA, 50mM NaF, 0.1 mM sodium vanadate, 1 mM phenylmethylsulfonic fluoride (PMSF), and 20 µg/ml aprotinin), and centrifuged at 14,000 rpm at 4°C for 10 minutes. To the supernatant (950 µl) was added 5 µl of anti-human p16 antibody to react for 1 hour for immunoprecipitation, and the immunoprecipitate was collected by Protein Sepharose A beads (Piace).
4) The beads were washed with 1 ml of the NP40 buffer 3 times and with 10 mM Tris-HCl (pH 7.5) once, suspended in 20 µl of 2 × sample buffer (1 × sample buffer is 62.5 mM Tris-HCl (pH 6.8), 5% 2-mercaptoethanol, 2% SDS, 10% glycerol, and 0.003% bromophenol blue), and boiled for 5 minutes. The resultant protein was fractionated by 12% SDS-PAGE electrophoresis (a conventional method).

As the positive and negative control, cdk4 and ΔE12 were used, respectively.

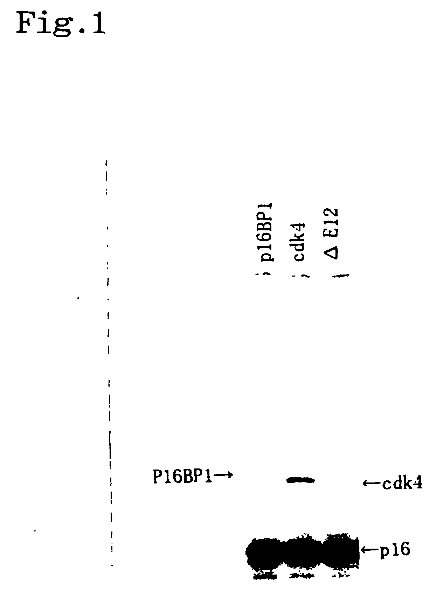

The photograph of the result of electrophoresis is shown in Fig. 1. The result of the sample of a mixture of p16 and p16BP1 is shown in the left lane, that of a mixture of p16 and cdk4 is shown in the middle lane, and that of a mixture of p16 and ΔE12 is shown in the right lane. In the immunoprecipitate with anti-human p16 antibody, human p16BP1 which bound to human p16 was detected in addition to human p16, and it was confirmed that human 16P binds to human p16BP1.

### 〈Example 3〉 Comparison of expression level of human p16BP1 gene in each tissue

A membrane plotted with 2 µg of each poly A⁺ RNA (mRNA) in various human tissues (Human Multiple Tissue Northern Blot II; Clonetech) was hybridized with labeled human p16BP1 cDNA in a hybridization buffer of the following composition, and analyzed by northern blot hybridization method.

### 1. Preparation of labeled probe

Labeling of human p16BP1 cDNA was carried out by using Megaprime Labeling Kit (RTM; Amersham) to label with ³²P-dCTP (Amersham).

### 2. Prehybridization

The aforementioned membrane was wetted with a hybridization buffer of the following composition, and placed in a sealable bag, and the bag was added with 10 ml of the hybridization buffer and sealed. Prehybridization was carried out at 42°C for 2 hours. The composition of the hybridization buffer was as follows.

| | |
|---|---|
| Formaldehyde | 25 ml |
| 20 × SSC | 12.5 ml |
| 0.5M sodium phosphate (pH 6.5) | 5 ml |
| Denatured salmon sperm DNA | 0.5 ml |
| 100 × Denhartd's | 2 ml |
| H₂O | 5 ml |
| Total | 50 ml |

### 3. Hybridization

One corner of the bag was cut off, the hybridization buffer was completely removed out, 10 ml of fresh hybridization buffer was added, the labeled probe previously boiled for 3 minutes (labeled p16BP1 cDNA) was added, and the bag was sealed. After hybridization at 42°C overnight, the bag was cut to open, and the hybridization buffer was completely removed. The membrane was taken out and rinsed with 2×SSC/0.1% SDS once. The membrane was repeatedly washed with 200 ml of 2×SSC/0.1% SDS (50°C) 3 times for 20 minutes each, and dried in air.

### 4. Exposure and development

The membrane was exposed with a X-ray film together with a sensitizing screen at -70°C overnight, and the X-ray film was developed.

### 5. Image analysis

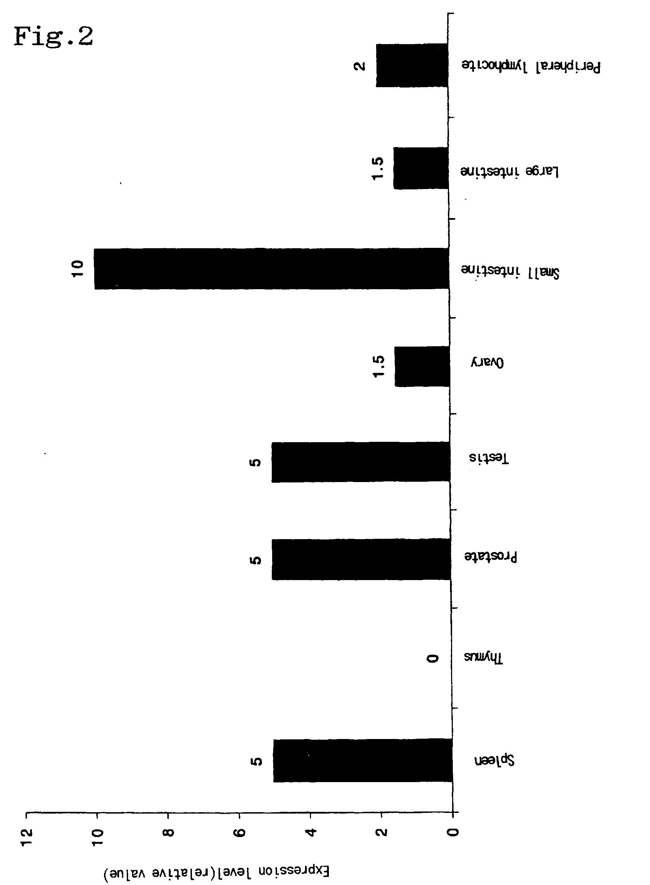

The X-ray film was scanned by a scanner (Epson, Model GT-6000), and the concentration of the band of p16BP1 mRNA of each tissue was numerically evaluated by using NIH Image (image analysis software provided by National Institutes of Health (NIH) of USA). The values obtained by the image analysis are shown in Fig. 2 as relative values. As shown in the figure, it was found that human p16BP1 mRNA expressed at the highest level in the small intestine and at extremely low level in the thymus gland among the normal tissues.

### 〈Example 4〉 Change of human p16BP1 mRNA level with the progression of cell cycle

Normal human fibroblast TIG3 (obtained from Tokyo Metropolitan Institute of Gerontology) was cultured in DMEM medium supplemented with 0.2% serum for 4 days, synchronized to the G0 phase (arrested growth condition) by serum starvation, and cultured in DMEM medium supplemented with 20% serum to progress the cell cycle by serum stimulation. The cell extract was prepared 0, 1, 2, 4, 8, 12, 16, 20, 24 and 30 hours after stimulation, respectively, and the total RNA was prepared from them, and northern blot hybridization was carried out by the following procedure using labeled human p16BP1 cDNA as a probe.

### 1. Preparation of labeled probe

Preparation was made as in Example 3.

### 2. Preparation of RNA filter

1.5 g of agarose and 15 ml of 10×MOPS were dissolved in 110 ml of sterilized MilliQ (RTM) water previously warmed to 80°C, and the mixture was then cooled to 60°C, and added with 25ml of formaldehyde. Well-stirred gel was applied to a submarine gel device (15 cm × 15 cm). Total RNA (20 µg) was added to 3.5 µl of sterilized MilliQ (RTM) water, and the mixture was placed in a sample tube of 15 ml volume. To this tube, 5 µl of formamide, 1.5 µl of 10×MOPS and 2 µl of formaldehyde were added, and the tube was allowed to stand at 65°C for 5 minutes, than placed on ice, and added with 3 µl of 5×dye-glycerol.

The thus prepared RNA sample was loaded on a gel, soaked in 1×MOPS, and subjected to electrophoresis at 100 V for 3 hours. The RNA on the gel was transferred to nitrocellulose filter, allowed to stand overnight, and placed in vacuum at 80°C for 2 hours to fix the RNA on the filter.

### 3. Prehybridization

Prehybridization was carried out as in Example 3.

### 4. Hybridization

Hybridization was carried out as in Example 3.

### 5. Exposure and development

Exposure and development were carried out as in Example 3.

### 6. Image analysis

Image analysis was carried out as in Example 3.

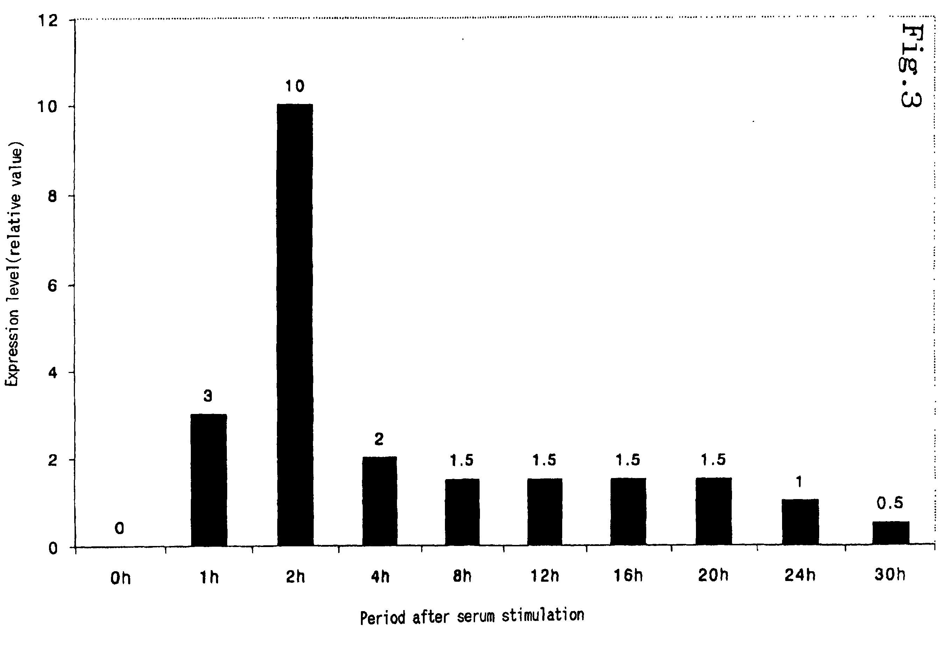

The values obtained by the image analysis are shown in Fig. 3 as relative values. As shown in the figure, it was found that human p16BP1 gene is not expressed under the arrested growth condition by serum starvation, and shows transient expression pattern where the expression starts 1 hour after growth induction, suddenly reaches at the peak after 2 hours and then rapidly decreases.

### 〈Example 5〉

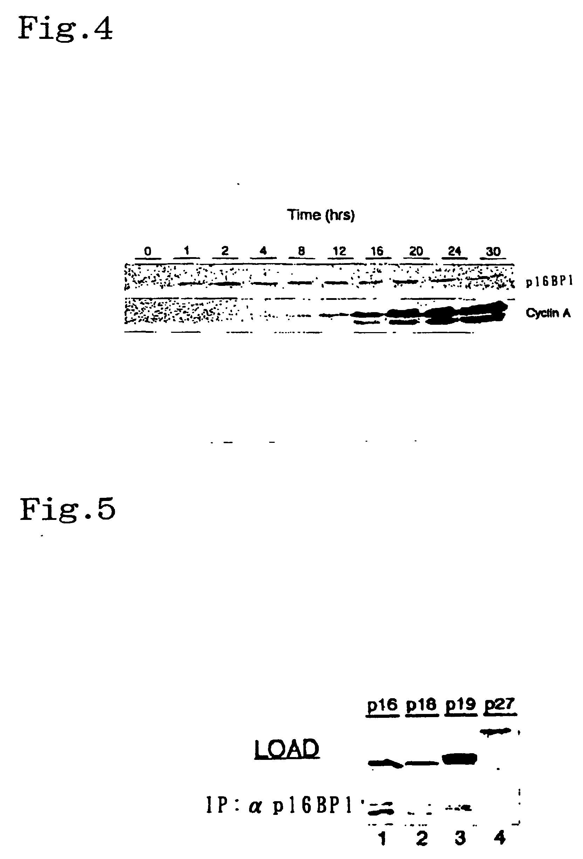

A peptide was prepared by adding Cys for coupling to the peptide sequence of p16BP1 (from Leu at the 222th to Arg at the 236th of amino acid sequence of SEQ ID No. 1 in the sequence listing) at the N-terminus, and rabbits was immunized with this peptide to obtain the antibody. Human normal fibroblasts TIG3 was cultured in DMEM medium supplemented with 0.2% serum for 4 days to be under the arrested growth condition, and then treated with DMEM medium supplemented with 20% serum for 0-30 hours to induce growth. The cell extract was prepared at a time shown in Fig. 4, respectively, and western blotting was carried out by using 150 µg of the extract to examine expression of p16BP1. In the examination, the antibody against p16BP1 obtained in the abovementioned way was used. As a control for growth induction, Cyclin A antibody (Santa Cruz H432) was used to carry out western blotting for Cyclin A protein, and the expression was observed. As a result, expression of p16BP1 was observed 1 hour after growth stimulation, and the level of expression was almost maintained after that.

### 〈Example 6〉

As in Example 2, p16BP1 having histidine tag (6 histidines) was expressed in E. coli, and expressed in vitro to examine interaction with p16, p18, p19 or p27 labeled with radioisotope. p16, p18, p19 or p27 was synthesized by using a TNT expression system according to the method recommended by the manufacturer (Promega) and continuously transcribing and translating plasmid DNA. The binding assay was carried out at 30°C for 30 minutes by using a mixture of unlabeled and ³⁵S-methionine labeled protein, or further using a protein having histidine tag purified from bacterial expression system (Parry, D. et al.: EMBO J. 14, 503-511, 1995). The reaction mixture was diluted with 1 ml of an ice-cold buffer containing 500 mM NaCl, 1% (v/v) NP4O and 3% (w/v) bovine serum albumin to perform immunoprecipitation with p16BP1 antibody prepared in Example 5. Fig. 5 represents results in the cases where both proteins were mixed and the sample was directly analyzed (Fig. 5, upper column, lane of LOAD), and where analyzed after immunoprecipitation with an antibody against p16BP1 (Fig. 5, lower column, lane of IP: α p16BP1). As a result, it was confined that p16BP1 interacts with p16, p18 and p19, but does not bind to p27.

### 〈Example 7〉

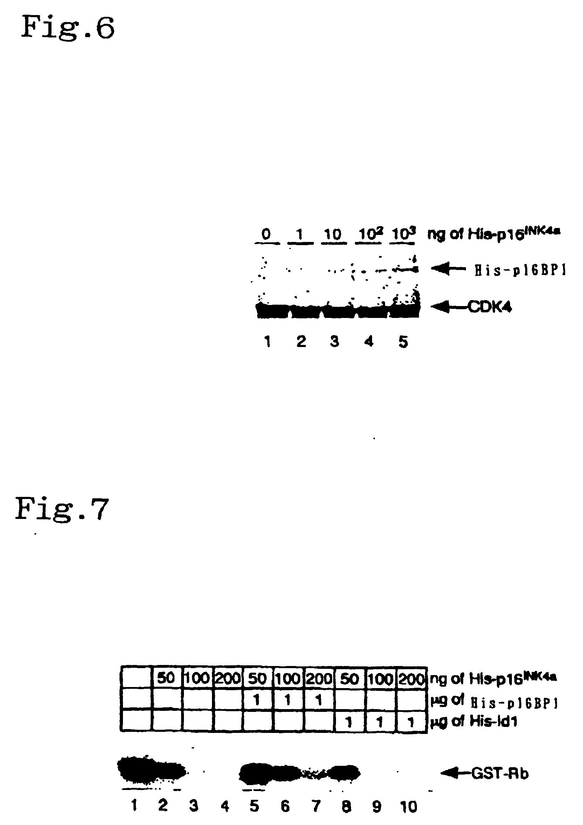

³⁵S labeled CDK4 and p16BP1 having histidine tag are mixed with p16 having a histidine tag, and the mixture was subjected to immuno-coprecipitation with anti-CDK4 antibody (Santa Cruz C-22), and analyzed by SDS-PAGE. The result is shown in Fig. 6. The more p16 added to the sample increased, the more p16BP1 collected together with CDK4 increased. The result suggests that p16BP1 does not inhibit the binding of p16 with CDK4, but forms a complex of p16BP1-p16-CDK4.

### 〈Example 8〉

p16 forms a complex with CDK4 to inhibit formation of CDK4-Cyclin D complex and decrease kinase activity of CDK4. The effect of p16BP1 on the action of p16 was examined. By using HPLC purified cyclin D1-CDK4 complex prepared from Baculovirus-infected sf9 cells, in vitro kinase assay was performed according to the method described in the literature (Matsushime, H., et al.: Mol. Cell. Biol. 14, 2066-2076, 1994; Kato, J., et al.: Genes Dev. 7, 331-342, 1993; Kitagawa, M., et al.: EMBO J. 15, 7060-7069, 1996). By using anti-CDK4 antibody (Santa Cruz C-22), CDK4 was immunoprecipitated from 3Y1 cells, and incubated with a substrate of GST-Rb protein (expressed in E. coli according to a conventional method and purified by a glutathione-agarose column) in the final volume of 25 ml of R buffer (20 mM Tris-HCl, pH 7.4, 10 mM MgCl₂, 4.5 mM 2-mercaptoethanol, 1 mM EGTA) containing 50 mM ATP and 10 µCi [γ-³²P]ATP (6000 Ci/mmol, Amersham). After treatment at 37°C for 30 minutes, the phosphorylated protein was subjected to SDS-PAGE and analyzed by autoradiography.

The result is shown in Fig. 7. Purified cyclin D1-CDK4 efficiently phosphorylated GST-Rb protein (lane 1). When p16 was added, the phosphorylation was inhibited (lane 2, 3 and 4). When p16BP1 was further added, the inhibition of phosphorylation by p16 was weakened (lane 5, 6 and 7). This effect was specific to p16BP1, and such effect was not recognized when unrelated protein was added (Id1: a controlling factor for cell growth, USP 5,527,897). The result above indicates that p16BP1 antagonizes the function of p16 to elevate the phosphorylating ability of CDK4 and thereby exert cell growth effect.

### 〈Example 9〉

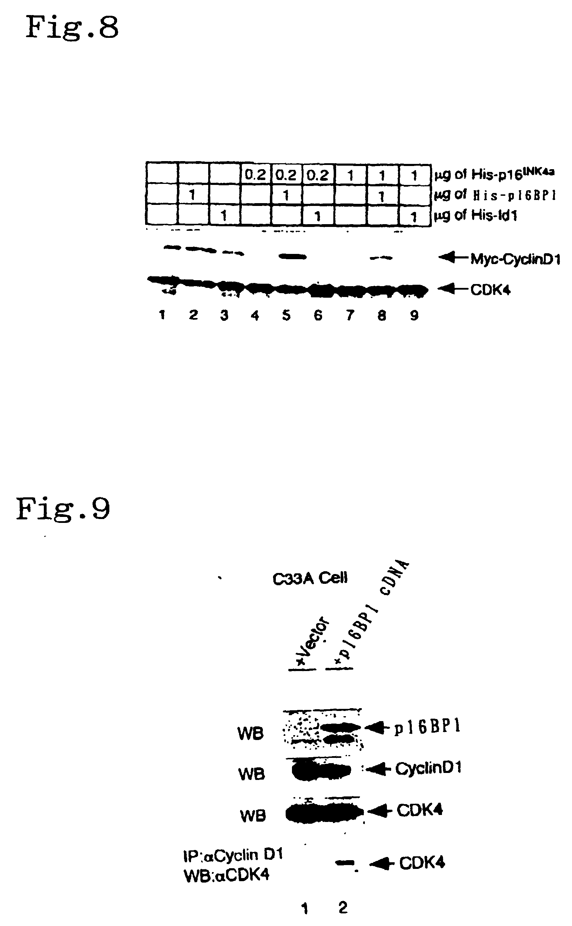

Using various combinations shown in Fig. 8, ³⁵S-labeled CDK4, Myc-labeled cyclin D1, histidine-labeled p16 expressed in E. coli, p16BP1, and Id1 were mixed. The sample was immunoprecipitated using anti-CDK4 antibody (Santa Cruz C-22) and analyzed by SDS-PAGE. The result is shown in Fig. 8. p16 inhibited the binding of Cyclin D1 with CDK4 by binding to CDK4 (lane 1, 4 and 7). When p16BP1 was added, inhibition of the binding by p16 was weakened (lane 4 and 5, lane 7 and 8). This effect was specific to p16BP1, and not recognized for unrelated protein (Id1) (lane 4 and 6, lane 7 and 9).

### 〈Example 10〉

C33A cells expressing p16 (obtained from ATCC) were transfected with p16BP1 expressing vector (lane 2) or vector alone (lane 1) (pCDNA3, Invitrogen). The cells were cultured in DMEM medium supplemented with 10% serum, and cell lysate was prepared 48 hours after the transfection and subjected to immunoblotting directly, or subjected to immunoprecipitation using antibody against Cyclin D1, and then western blotting using CDK4 antibody (Santa Cruz H303). The result is shown in Fig. 9. The upper three columns represent the result where the amounts of p16BP1, Cyclin D1 and CDK4 in each cell extract were confirmed by western blot. As primary antibodies, the antibody prepared in Example 5, anti-Cyclin D1 antibody (Santa Cruz R124), and anti-CDK4 antibody (Santa Cruz H303) were used, respectively. When p16BP1 was over-expressed (light lane), binding of Cyclin D1 and CDK4 which is not observed in normal C33A cells became observed (the bottom lane).

### 〈Example 11〉

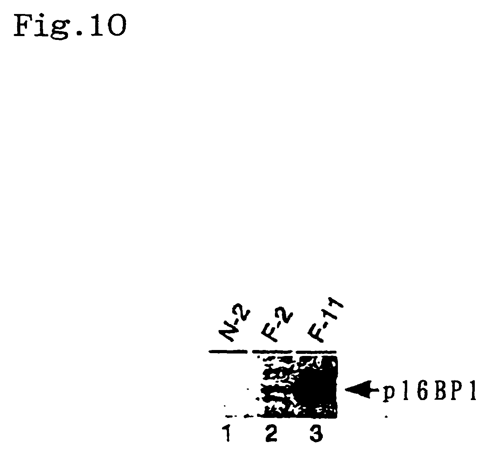

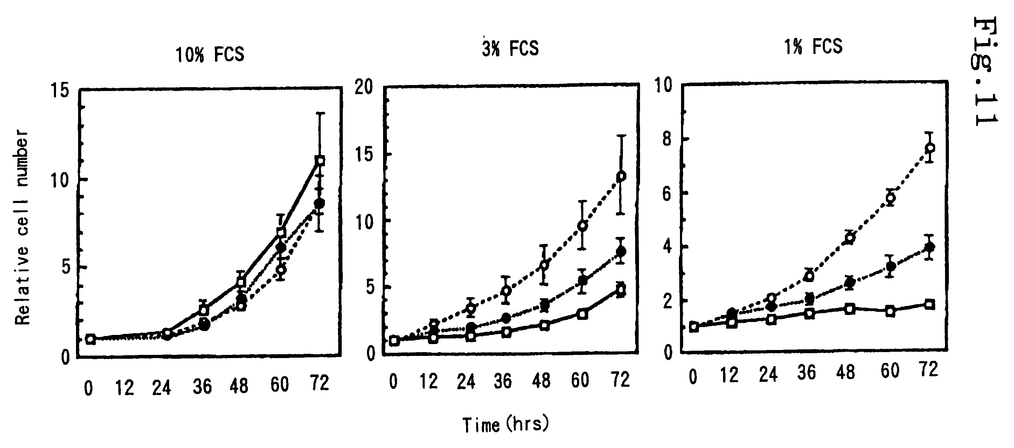

The action of p16BP1 on cell growth of rat fibroblast 3Y1 was examined. By using the FuGENE6 reaction mixture (Behringer Mannheim) according to the instruction, p16BP1 expression vector was transfected into 3Y1 cells expressing p16, and random sampling gave the strain expressing p16BP1 at low level (F-2) and that at high level (F-11) (Fig. 10, lane 2: F-2; lane 3: F-11; lane 1: N-2 cells transformed with vector alone). The extracts prepared from these stable transformants were subjected to immunoblotting using antibody against p16BP1. The strains, N-2, F-2 and F-11 were cultured in three kinds of medium, respectively, and the number of cell was counted at 12 hour intervals. The result is shown in Fig. 11 as relative values defining the value at the beginning of culture as 1 (In the figure, □, ●, and ○ represent the result of N-2, F-2, and F-11, respectively.). In DMEM medium supplemented with 10% serum, all of the parent strain (N-2), F-2 and F-11 showed similar growth. However, in DMEM medium supplemented with 3% or 1% serum, cell growth was F-11 > F-2 > N-2, showing that p16BP1 has positive effect on cell growth.

### Industrial Applicability

Human p16BP1 identified in the present invention binds to a tumor suppressor gene product p16 to antagonize p16 and stabilize interaction of cyclin D and CDK4, and thereby has a function of elevating phosphorylation of CDK4. Moreover, since cells expressing p16BP1 at high level have a high growth ability even under low serum conditions, it is strongly suggested that p16 exerts positive effect on cell growth. Furthermore, p16BP1 is located on the 19q13.1-13.2 of the human chromosome, and it is known that this region is amplified in various cancers. From these findings, functions of human p16BP1 seems to directly relate to the progression of the cell cycle, and to suppression of cancers.

Accordingly, information obtained from p16BP1 and its gene of the present invention can be utilized for the study of control of cell cycle and suppression of cancers, and for the treatment or diagnosis of diseases based on their findings. Moreover, the polynucleotide of the present invention can be used as a probe, and antisense polynucleotide or its derivatives are useful for inhibition of p16BP1 biosynthesis.

Furthermore, the method for obtaining cDNA which is the homologue of human p16BP1 cDNA by using the polynucleotide of the present invention provides p16BP1 cDNA and p16BP1 of various vertebrate animals, so that it becomes possible to study control of cell cycle and suppression of cancers, and treat or diagnose diseases based on their findings. The antibody of the present invention can be used for purification and detection of p16BP1.

## Claims

1. A protein which has an amino acid sequence of SEQ ID No.1 in the sequence listing.

2. A protein which has an amino acid sequence wherein one or more amino acids are deleted, substituted and/or added in an amino acid sequence of SEQ ID No. 1 in the sequence listing, and is capable of binding to p16.

3. A polynucleotide which encodes the protein of claims 1 or 2.

4. The polynucleotide of claim 3 which is an antisense polynucleotide or an derivative of said antisense polynucleotide.

5. The polynucleotide of claim 3 or 4 which consists of consecutive 12 or more bases.

6. The polynucleotide of any one of claims 3 to 5 which is chemically modified.

7. A method for obtaining cDNA which is a homologue of DNA having a base sequence of SEQ ID No.2 in the sequence listing, wherein the polynucleotide of claim 5 or 6 is used as a probe, and an cDNA capable of hybridizing with said probe is obtained from cDNA library of a vertebrate animal.

8. A cDNA of 650-780 bases which is a homologue of DNA having a base sequence of SEQ ID No.2 in the sequence listing, and is obtained by a method of claim 7.

9. A protein which is a homologue of protein of claim 1, and has an amino acid sequence encoded by cDNA of claim 8, and is capable of binding to p16.

10. An antibody which recognizes a protein of any one of claim 1, 2 and 9.
